# EUROPÄISCHE PATENTSCHRIFT

(11) **EP 1 630 166 B1**
(45) Veröffentlichungstag und Bekanntmachung des Hinweises auf die Patenterteilung: **03.02.2010**
(21) Anmeldenummer: 05024540.6
(22) Anmeldetag: 25.10.2000
(51) Int. Cl.: C07F 9/145, C07F 15/04, C07C 253/10, C07C 253/30, B01J 31/18

(54) **Nickel Komplexe mit Phosphitliganden**
Nickel Complexes with phosphite ligands
Complexes de nickel à ligands phosphite

(30) Priorität: 03.11.1999 DE 19953058
(43) Veröffentlichungstag der Anmeldung: 01.03.2006
(62) Teilanmeldung aus: 00979510.5
(73) Patentinhaber: BASF SE, 67056 Ludwigshafen (DE)
(72) Erfinder: Baumann, Robert, Dr., 68159 Mannheim (DE); Fischer, Jakob, Dr., 85414 Kirchdorf (DE); Jungkamp, Tim, Dr., 2950 Kapellen (BE); Kunsmann-Keitel, Pascale, Dagmar, Dr., 67117 Limburgerhof (DE); Siegel, Wolfgang, Dr., 67117 Limburgerhof (DE)

(56) Entgegenhaltungen:
- WO-A-97/36856
- DE-A- 2 237 703
- US-A- 3 766 237
- CHEMICAL ABSTRACTS, vol. 61, no. 1, 1964, Columbus, Ohio, US; abstract no.: 621f, ANGERT L.G.: "Synthesis of mixed esters of alpha-naphtylphosphorous acid and their inhibiting action in oxidizing rubbers" XP002161284 & ZH. PRIKL. KHIM., vol. 36, no. 10, 1963, pages 2270-2276,

## Beschreibung

Die vorliegende Erfindung betrifft neue Phosphite, Verfahren zu ihrer Herstellung, ihre Verwendung als Ligand in Übergangsmetallkomplexen, neue Übergangsmetallkomplexe, Verfahren zu ihrer Herstellung, ihre Verwendung als Katalysator und Verfahren in Gegenwart solcher Übergangsmetallkomplexe als Katalysator.

Triarylphosphite, Nickel-Komplexe mit solchen Phosphiten als Liganden und die Verwendung solcher Komplexe als Katalysator sind bekannt.

DE-OS 2 237 703, US-A-3,850,973 und US-A-3,903,120 beschreiben ein Verfahren zur Hydrocyanierung von ungesättigten organischen Verbindungen und die Isomerisierung von Nitrilen in Anwesenheit von Nickel(0)-Komplexen mit Tri-o-tolyl-phosphit als Ligand. Nachteilig bei diesem Verfahren ist, dass die Stabilität solcher Nickel-Komplexe unbefriedigend ist. Diese geringe Stabilität zeigt sich in einem sehr geringen Gehalt an Ni(0), welches für die Hydrocyanierung die aktive Spezies ist, in der Reaktionslösung.

US-A-3,766,237 und US-A-3,903,120 beschreiben ein Verfahren zur Hydrocyanierung von ungesättigten organischen Verbindungen und die Isomerisierung von Nitrilen in Anwesenheit von Nickel(0)-Komplexen mit Tri-m/p-tolyl-phosphit als Ligand. Nachteilig bei diesem Verfahren ist, dass die Reaktivität solcher Nickel-Komplexe unbefriedigend ist.

Es war daher die technische Aufgabe gestellt, ein Verfahren zur Verfügung zu stellen, das die Hydrocyanierung von ungesättigten organischen Verbindungen bei hoher Stabilität und hoher Reaktivität des Katalysators auf technisch einfache und wirtschaftliche Weise ermöglicht.

Demgemäß wurde ein neues Verfahren zur Herstellung von Übergangsmetallkomplexen gefunden, welches dadurch gekennzeichnet ist, dass man Ni(0) oder eine Ni(0) enthaltende chemische Verbindung mit einem Phosphit der Formel I

P (O-R¹)ₓ (O-R²)_{y} (O-R³)_{z} (O-R⁴)ₚ I

mit a)
- R¹:: aromatischer Rest mit einem C₁-C₁₈-Alkylsubstitutenten in o-Stellung zu dem Sauerstoffatom, das das Phosphoratom mit dem aromatischen Rest verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem n-Propyl-, i-Propyl-, n-Butyl-, i-Butyl-, s-Butyl-, n-Pentylrest sowie deren Isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt.
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt.
oder
mit b)
- R¹:: aromatischer Rest mit einem C₁-C₁₈-Alkylsubstitutenten in o-Stellung zu dem Sauerstoffatom, das das Phosphoratom mit dem aromatischen Rest verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt.
- R³:: aromatischer Rest mit einem n-Propyl-, i-Propyl-, n-Butyl-, i-Butyl-, s-Butyl-, n-Pentylrest sowie deren isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt, und für a) und b) gilt:
- x :: 1 oder 2,
- y, z, p:: unabhängig voneinander 0, 1 oder 2 mit der Maßgabe, dass x + y + z + p = 3,
umsetzt,
neue Übergangsmetallkomplexe, ihre Verwendung als Katalysator und Verfahren in Gegenwart solcher Übergangsmetallkomplexe als Katalysator gefunden.

Erfindungsgemäß ist der Rest R¹ ein aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System.

Als aromatischer Rest kommen Heterocyclen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen C₁-C₁₈-Alkylsubstituenten in o-Stellung zu dem Sauerstoffatom, das das Phosphoratom mit dem aromatischen System verbindet oder ein in o-Stellung zu dem Sauerstoffatom, das das Phosphoratom mit dem aromatischen System verbindet, anelliertes aromatisches System. Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

### a)

Erfindungsgemäß ist der Rest R² ein aromatischer Rest mit einem n-Propyl-, i-Propyl-, n-Butyl-, i-Butyl-, s-Butyl-, n-Pentylrest sowie deren Isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt.

Als aromatischer Rest kommen Heterocyclen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten in m-Stellung zu dem Sauerstoffatom, das das Phosphoratom mit dem aromatischen System verbindet oder ein in m-Stellung zu dem Sauerstoffatom, das das Phosphoratom mit dem aromatischen System verbindet, anelliertes aromatisches System. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten.können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R² kommen vorteilhaft m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen in Betracht.

Erfindungsgemäß ist der Rest R³ ein aromatischer Rest mit einem C₁-C₁₈-Akyl-substituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.

Als aromatischer Rest kommen Heterocyclen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen C₁-C₁₈-Alkylsubstituenten in p-Stellung zu dem Sauerstoffatom, das das Phosphoratom mit dem aromatischen System verbindet oder ein in p-Stellung zu dem Sauerstoffatom, das das Phosphoratom mit dem aromatischen System verbindet, anelliertes aromatisches System. Als Alkylrest können lineare oder cyclische C₁- bis C₁₈-Reste, vorzugsweise C₁-C₉-Reste eingesetzt werden, wie Methyl-, Ethyl-, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise'trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Erfindungsgemäß ist der Rest R⁴ ein aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt.

Der aromatische Rest kann funktionelle Gruppen tragen, wie Alkoxy-Gruppen oder Halogene, beispielsweise Chlor oder Brom, tragen, vorzugsweise trägt der aromatische Rest keine funktionellen Gruppen.

### b)

Erfindungsgemäß ist der Rest R² ein aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt.

Als aromatischer Rest kommen Heterocyclen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß einen C₁-C₁₈-Alkylsubstituenten in m-Stellung zu dem Sauerstoffatom, das das Phosphoratom mit dem aromatischen System verbindet oder ein in m-Stellung zu dem Sauerstoffatom, das das Phosphoratom mit dem aromatischen System verbindet, anelliertes aromatisches System. Als Alkylrest können lineare oder cyclische C₁- bis C₁₈-Reste, vorzugsweise C₁-C₉-Reste eingesetzt werden, wie Methyl-, Ethyl-, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R² kommen vorteilhaft m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen in Betracht.

Erfindungsgemäß ist der Rest R³ ein aromatischer Rest mit einem n-Propyl-, i-Propyl-, n-Butyl-, i-Butyl-, s-Butyl-, n-Pentylrest sowie deren Isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.

Als aromatischer Rest kommen Heterocyclen, vorzugsweise Homocyclen, wie der Phenylrest in Betracht.

Der aromatische Rest kann weitere funktionelle Gruppen tragen, wie Alkoxy-Gruppen oder Halogene, beispielsweise Chlor oder Brom, tragen, vorzugsweise trägt der aromatische Rest keine funktionellen Gruppen.

Der aromatische Rest trägt erfindungsgemäß n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten in p-Stellung zu dem Sauerstoffatom, das das Phosphoratom mit dem aromatischen System verbindet oder ein in p-Stellung zu dem Sauerstoffatom, das das Phosphoratom mit dem aromatischen System verbindet, anelliertes aromatisches System. Bevorzugte Alkylreste sind n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als aromatischer Substituent kommen Heterocyclen, vorzugsweise Homocyclen, wie der Phenylrest in Betracht.

Der aromatische Substituent kann weitere funktionelle Gruppen tragen, wie Alkoxy-Gruppen oder Halogene, beispielsweise Chlor oder Brom, tragen, vorzugsweise trägt der aromatische Substituent keine funktionellen Gruppen.

Der aromatische Substituent kann einen oder mehrere C₁-C₁₈-Alkylsubstituenten oder eines oder mehrere anellierte aromatische Systeme tragen oder frei von weiteren Substituenten sein.

Als Alkylrest können lineare oder cyclische C₁- bis C₁₈-Reste, vorzugsweise C₁-C₉-Reste eingesetzt werden, wie Methyl-, Ethyl, n-Propyl-, i-Propyl-, n-Butyl-, i-Butyl-, s-Butyl-, n-Pentylreste sowie deren Isomeren, n-Hexylreste sowie deren Isomeren, Cyclopentyl- oder Cyclohexylreste, wobei die cyclischen Alkylreste lineare oder weitere cyclische Alkylreste oder aromatische Reste und die Alkylreste cyclische Alkylreste oder aromatische Reste als Substituenten tragen können. Bevorzugte Alkylreste sind Methyl-, Ethyl-, n-Propyl-, i-Propyl-, n-Butyl-, i-Butyl-, s-Butyl-Gruppen.

Diese Alkylreste können weitere funktionelle Gruppen tragen, wie Alkoxygruppen, Aminogruppen, wie unsubstituierte, mono- oder disubstituierte Aminogruppen, Mercaptogruppen, wie substituierte Mercaptogruppen, wobei die Substitution durch die genannten Alkylgruppen oder aromatischen Reste erfolgen kann. Vorzugsweise tragen die Alkylreste keine funktionellen Gruppen.

In den Alkylresten können Kohlenstoffatome durch andere Atome, wie Sauerstoff, Stickstoff oder Schwefel, substituiert sein, vorzugsweise sind die Alkylreste nicht substituiert.

Als Rest R³ kommen vorteilhaft p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Erfindungsgemäß ist der Rest R⁴ ein aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt.

Der aromatische Rest kann funktionelle Gruppen tragen, wie Alkoxy-Gruppen oder Halogene, beispielsweise Chlor oder Brom, tragen, vorzugsweise trägt der aromatische Rest keine funktionellen Gruppen.

Als Rest R⁴ kommt vorzugsweise der Phenylrest in Betracht.

Erfindungsgemäß beträgt der Index x 1 oder 2.

Erfindungsgemäß betragen die Indizes y, z und p unabhängig voneinander 0, 1 oder 2 mit der Maßgabe, dass die Summe der Indizes x, y, z und p, also x+y+z+p, 3 ist.

Vorzugsweise ist p=0.

Hieraus ergeben sich folgende erfindungsgemäße Möglichkeiten für die Indizes x, y, z und p:

| x | y | Z | P |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Besonders bevorzugte Phosphite sind solche, in denen R¹ der o-Tolylrest, R² der 2-Naphthylrest und R³ der p-Tolylrest ist mit den in der Tabelle genannten Indizes, solche, denen R¹ der o-Isopropylrest, R² der 2-Naphthylrest und R³ der p-Tolylrest ist mit den in der Tabelle genannten Indizes sowie Gemische dieser Phosphite.

Phosphite der Formel I können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I.

Die Umsetzung kann in drei getrennten Schritten durchgeführt werden.

Es können zwei der drei Schritte kombiniert werden, also a mit b oder b mit c.

Es können alle der Schritte a, b und c miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃.

In den Schritten a, b und c kann man die Umsetzung vorteilhaft bei Temperaturen im Bereich von 10 bis 200°C, vorzugsweise 50 bis 150°C, insbesondere 70 bis 120°C durchführen.

In den Schritten a, b und c kommt vorzugsweise ein molares Verhältnis zwischen den in den jeweiligen Schritten eingesetzten Halogenidresten und Hydroxylgruppen der eingesetzten Alkohole im Bereich von 1 : 10 bis 10 : 1, vorzugsweise 1 : 3 bis 3 : 1, in Betracht.

Die Umsetzung in den Schritten a, b und c kann man in Gegenwart eines anorganischen oder organischen, insbesondere flüssigen, Verdünnungsmittels durchführen, wie eines Esters, beispielsweise Essigsäureethylester, eines Ethers, beispielsweise Methyl-t-butylether, Diethylether, Dioxan, Tetrahydrofuran, einer aromatischen Verbindung, beispielsweise Toluol, oder eines halogenierten Kohlenwasserstoffs, beispielsweise halogenierten Kohlenwasserstoffs, wie Tetrachlormethan, Chloroform, Methylenchlorid, oder eines Gemisches solcher Verdünnungsmittel.

Vorzugsweise führt man die Umsetzung ohne ein solches anorganisches oder organisches Verdünnungsmittel durch.

Den bei der Umsetzung entstehenden Halogenwasserstoff, der unter den Reaktionsbedingungen üblicherweise gasförmig anfällt, kann vorteilhaft abgetrennt und an sich bekannten chemischen Verfahren zugeführt werden.

Üblicherweise erhält man in den Schritten a, b und c Mischungen, die die gewünschte Komponente enthalten.

Die Abtrennung der gewünschten Komponente kann in an sich bekannter Weise, beispielsweise durch Extraktion oder Destillation, vorzugsweise durch Destillation erfolgen.

Erfolgt die Abtrennung durch Destillation, so hat sich eine Verminderung des Drucks unter Umgebungsdruck als vorteilhaft erwiesen.

Die Destillation kann vorteilhaft in einer Kolonne, beispielsweise mit Seitenabzug, oder mehreren, wie zwei, drei, oder vier Kolonnen erfolgen.

Als Kolonnen können dabei an sich bekannte Kolonnen, wie Glockenbodenkolonnen, Siebbodenkolonnen oder Füllkörperkolonnen eingesetzt werden.

Die zur Abtrennung der entsprechenden Phosphite der Formel I optimalen Verfahrensbedingungen können dabei jeweils durch wenige einfache Vorversuche leicht ermittelt werden.

Die Phosphite der Formel I können als Liganden in Übergangsmetallkomplexen eingesetzt werden.

Als Übergangsmetalle kommen dabei vorteilhaft die Metalle der 1. bis 2. und 6. bis 8. Nebengruppe des Periodensystems, vorzugsweise der 8. Nebengruppe des Periodensystems, besonders bevorzugt Eisen, Kobalt und Nickel, insbesondere Nickel in Betracht.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Zur Herstellung der Übergangsmetallkomplexe kann man eine ein Übergangsmetall enthaltende chemische Verbindung oder vorzugsweise ein Übergangsmetall mit einem Phosphit der Formel I um, wobei als Phosphit der Formel I ein einzelnes Phosphit der Formel I oder ein Gemisch mehrerer Phosphite der Formel I eingesetzt werden kann.

Das Übergangsmetall kann vor der Umsetzung aus geeigneten chemischen Verbindungen, beispielsweise durch Reduktion mit unedlen Metallen wie Zink, aus Salzen, wie Chloriden, erhalten werden.

Setzt man zur Herstellung der Übergangsmetallkomplexe eine ein Übergangsmetall enthaltende Verbindung ein, so kommen hierzu vorteilhaft Salze, wie Chloride, Bromide, Acetylacetonate, Sulfate, Nitrate, beispielsweise Nickel(2)-Chlorid, in Betracht.

Nach der Umsetzung der ein Übergangsmetall enthaltenden Verbindung oder des Übergangsmetalls mit einem Phosphit der Formel I kann die Wertigkeit des Übergangsmetalls in dem Komplex mit geeigneten Oxidations- oder Reduktionsmitteln, beispielsweise unedlen Metallen, wie Zink, oder Wasserstoff in chemisch gebundener Form, wie Natriumborhydrid, oder in molekularer Form, oder elektrochemisch verändert werden.

In den Übergangsmetallkomplexen kann das molare Verhältnis von Übergangsmetall zu Phosphit der Formel I im Bereich zwischen 1 und 6, vorzugsweise 2 bis 5, insbesondere 2, 3 oder 4 betragen.

Die Übergangsmetallkomplexe können frei von anderen Liganden als den Phosphiten der Formel I sein.

Die Übergangsmetallkomplexe können neben den Phosphiten der Formel I weitere Liganden enthalten, beispielsweise Nitrile, wie Acetonitril, Adipodinitril, 3-Pentennitril, 4-Pentennitril, 2-Methyl-3-butennitril, Olefine, wie Butadien.

Die Herstellung solcher Übergangsmetallkomplexe kann grundsätzlich in einer solchen Weise erfolgen, wie sie in der Literatur, beispielsweise in DE-OS-2 237 703, US-A-3,850,973, US-A-3,766,237 oder US-A-3,903,120, zur Herstellung von Übergangsmetallkomplexen, die Tri-o-tolyl-phosphit, Tri-m-tolyl-phosphit oder Tri-p-tolyl-phosphit enthalten, beschrieben ist, indem man diese Phosphite durch die erfindungsgemäßen Phosphite der Formel I teilweise oder vollständig ersetzt.

Die erfindungsgemäßen Übergangsmetallkomplexe können als Katalysator, insbesondere als Homogenkatalysator eingesetzt werden.

Als besonders vorteilhaft hat sich die Verwendung der erfindungsgemäßen Übergangsmetallkomplexe als Katalysator bei der Addition von Blausäure an olefinische Doppelbindungen, insbesondere solche, die in Konjugation zu einer weiteren olefinischen Doppelbindung stehen, beispielsweise von Butadien unter Erhalt einer Mischung enthaltend 2-Methyl-3-butennitril und 3-Pentennitril, erwiesen. Gleichermaßen vorteilhaft ist auch die Verwendung als Katalysator bei der Addition von Blausäure an olefinische Doppelbindungen, die nicht in Verbindung mit einer weiteren olefinischen Doppelbindung stehen, beispielsweise von 3-Pentennitril oder 4-Pentennitril oder deren Gemische, vorzugsweise 3-Pentennitril, unter Erhalt von Adipodinitril, oder von 3-Pentensäureester oder 4-Pentensäureester oder deren Gemische, vorzugsweise 3-Pentensäureester, unter Erhalt von 5-Cyanovaleriansäureester.

Ebenso als besonders vorteilhaft hat sich die Verwendung der erfindungsgemäßen Übergangsmetallkomplexe als Katalysator bei der Isomerisierung organischer Nitrile, insbesondere solcher, bei denen die Nitrilgruppe nicht in Konjugation zu einer olefinischen Doppelbindung steht, beispielsweise von 2-Methyl-3-butennitril unter Erhalt von 3-Pentennitril, erwiesen. Gleichermaßen vorteilhaft ist auch die Verwendung als Katalysator bei der Isomerisierung organischer Nitrile, bei denen die Nitrilgruppe in Konjugation zu einer olefinischen Doppelbindung steht.

Verfahren zur Addition von Blausäure an eine olefinische Doppelbindung oder zur Isomerisierung von organischen Nitrilen kann grundsätzlich in einer solchen Weise erfolgen, wie sie in der Literatur unter Verwendung von Übergangsmetallkomplexen, die Tri-o-tolyl-phosphit, Tri-m-tolyl-phosphit oder Tri-p-tolyl-phosphit enthalten, beschrieben ist, indem man diese Phosphite durch die erfindungsgemäßen Phosphite der Formel I teilweise oder vollständig ersetzt.

## Patentansprüche

1. Verfahren zur Herstellung von Übergangsmetallkomplexen, **dadurch gekennzeichnet, dass** man Ni(0) oder eine Ni(0) enthaltende chemische Verbindung mit einem Phosphit der Formel I
P (O-R¹)ₓ (O-R²)_{y} (O-R³)_{z} (O-R⁴)ₚ I
mit a)
R¹: aromatischer Rest mit einem C₁-C₁₈-Alkylsubstitutenten in o-Stellung zu dem Sauerstoffatom, das das Phosphoratom mit dem aromatischen Rest verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, annelierten aromatischen System,
R²: aromatischer Rest mit einem n-Propyl-, i-Propyl-, n-Butyl-, i-Butyl-, s-Butyl-, n-Pentylrest sowie deren Isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, annelierten aromatischen System, wobei der aromatische Rest in o-Stellung zu dem Sauerstoffatom, das das Phosphoratom mit dem aromatischen System verbindet, ein Wasserstoffatom trägt.
R³: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.
R⁴: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt.
oder mit b)
R¹: aromatischer Rest mit einem C₁-C₁₈-Alkylsubstitutenten in o-Stellung zu dem Sauerstoffatom, das das Phosphoratom mit dem aromatischen Rest verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, annelierten aromatischen System,
R²: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, annelierten aromatischen System, wobei der aromatische Rest in o-Stellung zu dem Sauerstoffatom, das das Phosphoratom mit dem aromatischen System verbindet, ein Wasserstoffatom trägt.
R³: aromatischer Rest mit einem n-Propyl-, i-Propyl-, n-Butyl-, i-Butyl-, s-Butyl-, n-Pentylrest sowie deren Isomeren, n-Hexylrest sowie deren Isomeren, Cyclopentyl- oder Cyclohexylrest, wobei die cyclischen Alkylreste lineare oder weitere cyclische Alkylreste oder aromatische Reste und die Alkylreste cyclische Alkylreste oder aromatische Reste als Substituenten tragen können, in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt.
R⁴: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt, und für a) und b) gilt:
x : 1 oder 2,
y, z, p: unabhängig voneinander 0, 1 oder 2 mit der Maßgabe,
dass x + y + z + p = 3,
umsetzt.

2. Verfahren nach Anspruch 1, wobei man ein Phosphit der Formel I mit p = 0 einsetzt.

3. Verfahren nach Anspruch 1 bis 2, wobei man ein Phosphit der Formel I, wobei die Reste R¹, R², R³ und R⁴ unabhängig voneinander ausgewählt sind aus der Gruppe bestehend aus Naphthylrest und gemäß Anspruch 1 substituiertem oder unsubstituiertem Phenylrest, einsetzt.

4. Verfahren nach den Ansprüchen 1 bis 3, wobei man ein Phosphit der Formel I, wobei die Reste R¹, R², R³ und R⁴ substituierte oder unsubstituierte Phenylreste sind, einsetzt.

5. Übergangsmetallkomplexe erhältlich gemäß den Ansprüchen 1 bis 4 mit der Maßgabe, dass y und z für 1 oder 2 stehen und p = 0 ist.

6. Verwendung von Übergangsmetallkomplexen gemäß Anspruch 5 als Katalysator.

7. Verwendung nach Anspruch 6 als Katalysator für die Addition von Blausäure an eine olefinische Doppelbindung.

8. Verwendung nach Anspruch 6 als Katalysator für die Isomerisierung organischer Nitrile.

9. Verfahren zur Addition von Blausäure an eine olefinische Doppelbindung in Gegenwart eines Katalysators, wobei man als Katalysator einen Übergangsmetallkomplex gemäß Anspruch 5 einsetzt.

10. Verfahren nach Anspruch 9, wobei man Blausäure an Butadien addiert unter Erhalt einer Verbindung ausgewählt aus der Gruppe bestehend aus 2-Methyl-3-butennitril und 3-Pentennitril.

11. Verfahren zur Isomerisierung organischer Nitrile in Gegenwart eines Katalysators, wobei man als Katalysator einen Übergangsmetallkomplex gemäß Anspruch 5 einsetzt.

12. Verfahren nach Anspruch 11, wobei man 2-Methyl-3-butennitril zu 3-Pentennitril isomerisiert.

## Claims

1. A process for preparing transition metal complexes, which comprises reacting Ni (O) or an Ni (O) - comprising chemical compound with a phosphite of the formula I
P (O-R¹)ₓ (O-R²)_{y} (O-R³)_{z} (O-R⁴)ₚ I
where a)
R¹: aromatic radical having a C₁-C₁₈-alkyl substituent in the o position relative to the oxygen atom which connects the phosphorus atom to the aromatic radical, or having an aromatic substituent in the o position relative to the oxygen atom which connects the phosphorus atom to the aromatic system, or having an aromatic system fused on in the o position relative to the oxygen atom which connects the phosphorus atom to the aromatic system,
R² aromatic radical having an n-propyl, i-propyl, n-butyl, i-butyl, s-butyl, n-pentyl radical and its isomers, n-hexyl radical and its isomers, cyclopentyl or cyclohexyl radical, where the cyclic alkyl radicals may bear linear or further cyclic alkyl radicals or aromatic radicals as substituents and the alkyl radicals may bear cyclic alkyl radicals or aromatic radicals as substituents, in the m position relative to the oxygen atom which connects the phosphorus atom to the aromatic system, or having an aromatic substituent in the m position relative to the oxygen atom which connects the phosphorus atom to the aromatic system, or having an aromatic system fused on in the m position relative to the oxygen atom which connects the phosphorus atom to the aromatic system, where the aromatic radical bears a hydrogen atom in the o position relative to the oxygen atom which connects the phosphorus atom to the aromatic system,
R³: aromatic radical having a C₁-C₁₈-alkyl substituent in the p position relative to the oxygen atom which connects the phosphorus atom to the aromatic system, or having an aromatic substituent in the p position relative to the oxygen atom which connects the phosphorus atom to the aromatic system, where the aromatic radical bears a hydrogen atom in the o position relative to the oxygen atom which connects the phosphorus atom to the aromatic system,
R⁴: aromatic radical which bears substituents other than those defined for R¹, R² and R³ in the o, m and p positions relative to the oxygen atom which connects the phosphorus atom to the aromatic system, where the aromatic radical bears a hydrogen atom in the o position relative to the oxygen atom which connects the phosphorus atom to the aromatic system,
or
where b)
R¹: aromatic radical having a C₁-C₁₈-alkyl substituent in the o position relative to the oxygen atom which connects the phosphorus atom to the aromatic radical, or having an aromatic substituent in the o position relative to the oxygen atom which connects the phosphorus atom to the aromatic system, or having an aromatic system fused on in the o position relative to the oxygen atom which connects the phosphorus atom to the aromatic system,
R²: aromatic radical having a C₁-C₁₈-alkyl substituent in the m position relative to the oxygen atom which connects the phosphorus atom to the aromatic system, or having an aromatic substituent in the m position relative to the oxygen atom which connects the phosphorus atom to the aromatic system, or having an aromatic system fused on in the m position relative to the oxygen atom which connects the phosphorus atom to the aromatic system, where the aromatic radical bears a hydrogen atom in the o position relative to the oxygen atom which connects the phosphorus atom to the aromatic system,
R³: aromatic radical having an n-propyl, i-propyl, n-butyl, i-butyl, s-butyl, n-pentyl radical and its isomers, n-hexyl radical and its isomers, cyclopentyl or cyclohexyl radical, where the cyclic alkyl radicals may bear linear or further cyclic alkyl radicals or aromatic radicals as substituents, in the p position relative to the oxygen atom which connects the phosphorus atom to the aromatic system, or having an aromatic substituent in the p position relative to the oxygen atom which connects the phosphorus atom to the aromatic system, where the aromatic radical bears a hydrogen atom in the o position relative to the oxygen atom which connects the phosphorus atom to the aromatic system,
R⁴ aromatic radical which bears substituents other than those defined for R¹, R² and R³ in the o, m and p positions relative to the oxygen atom which connects the phosphorus atom to the aromatic system, where the aromatic radical bears a hydrogen atom in the o position relative to the oxygen atom which connects the phosphorus atom to the aromatic system, and for a) and b):
x : 1 or 2,
y, z, p: independently of one another, 0, 1 or 2, with the proviso that x+y+z+p = 3.

2. The process according to claim 1, wherein a phosphite of the formula I in which p = 0 is used.

3. The process according to claim 1 or 2, wherein a phosphite of the formula I in which the radicals R¹, R², R³ and R⁴ are selected independently from the group consisting of a naphthyl radical and a phenyl radical which is unsubstituted or substituted according to claim 1 is used.

4. The process according to any of claims 1 to 3, wherein a phosphite of the formula I in which the radicals R¹, R², R³ and R⁴ are substituted or unsubstituted phenyl radicals is used.

5. A transition metal complex which can be obtained according to any of claims 1 to 4, with the proviso that y and z are each 1 or 2 and p = 0.

6. The use of transition metal complexes according to claim 5 as catalyst.

7. The use according to claim 6 as catalyst for the addition of hydrocyanic acid onto an olefinic double bond.

8. The use according to claim 6 as catalyst for the isomerization of organic nitriles.

9. A process for the addition of hydrocyanic acid onto an olefinic double bond in the presence of a transition metal complex according to claim 5 as catalyst.

10. The process according to claim 9, wherein hydrocyanic acid is added onto butadiene to give a compound selected from the group consisting of 2-methyl-3-butenenitrile and 3-pentenenitrile.

11. A process for the isomerization of organic nitriles in the presence of a transition metal complex according to claim 5 as catalyst.

12. The process according to claim 11, wherein 2-methyl-3-butenenitrile is isomerized to 3-pentenenitrile.

## Revendications

1. Procédé pour la préparation de complexes de métaux de transition, **caractérisé en ce qu'**on transforme du Ni(0) ou un composé chimique contenant du Ni(0) avec un phosphite de formule I
P(O-R¹)ₓ(O-R²)_{y} (O-R³)_{z}(O-R⁴)ₚ I
avec a)
R¹ : signifiant un radical aromatique avec un substituant C₁-C₁₈-alkyle en position ortho par rapport à l'atome d'oxygène, qui lie l'atome de phosphore au radical aromatique, ou avec un substituant aromatique en position ortho par rapport à l'atome d'oxygène qui relie l'atome de phosphore au système aromatique ou avec un système aromatique annelé en position ortho par rapport à l'atome d'oxygène qui lie l'atome de phosphore au système aromatique,
R² : signifiant un radical aromatique avec un radical n-propyle, i-propyle, n-butyle, i-butyle, s-butyle, n-pentyle ainsi que ses isomères, un radical n-hexyle ainsi que ses isomères, un radical cyclopentyle ou cyclohexyle, où les radicaux alkyle cycliques peuvent porter des radicaux alkyle linéaires ou d'autres radicaux alkyle cycliques ou des radicaux aromatiques et les radicaux alkyle peuvent porter des radicaux alkyle cycliques ou des radicaux aromatiques comme substituants, en position méta par rapport à l'atome d'oxygène qui lie l'atome de phosphore au système aromatique, ou avec un substituant aromatique en position méta par rapport à l'atome d'oxygène qui lie l'atome de phosphore au système aromatique ou avec un système aromatique annelé en position méta par rapport à l'atome d'oxygène qui lie l'atome de phosphore au système aromatique, où le radical aromatique en position ortho par rapport à l'atome d'oxygène qui lie l'atome de phosphore au système aromatique porte un atome d'hydrogène,
R³ : signifiant un radical aromatique avec un substituant C₁-C₁₈-alkyle en position para par rapport à l'atome d'oxygène qui lie l'atome de phosphore au système aromatique ou avec un substituant aromatique en position para par rapport à l'atome d'oxygène qui lie l'atome de phosphore au système aromatique, ou le radical aromatique en position ortho par rapport à l'atome d'oxygène qui lie l'atome de phosphore au système aromatique, porte un atome d'hydrogène,
R⁴ : signifiant un radical aromatique qui porte, en position ortho, méta et para par rapport à l'atome d'oxygène qui lie l'atome de phosphore au système aromatique, d'autres substituants que ceux définis pour R¹, R² et R³, où le radical aromatique en position ortho par rapport à l'atome d'oxygène qui lie l'atome de phosphore au système aromatique, porte un atome d'hydrogène,
ou
avec b)
R¹ : signifiant un radical aromatique avec un substituant C₁-C₁₈-alkyle en position ortho par rapport à l'atome d'oxygène, qui lie l'atome de phosphore au radical aromatique, ou avec un substituant aromatique en position ortho par rapport à l'atome d'oxygène qui relie l'atome de phosphore au système aromatique ou avec un système aromatique annelé en position ortho par rapport à l'atome d'oxygène qui lie l'atome de phosphore au système aromatique,
R²: signifiant un radical aromatique avec un substituant C₁-C₁₈-alkyle en position méta par rapport à l'atome d'oxygène, qui lie l'atome de phosphore au système aromatique, ou avec un substituant aromatique en position méta par rapport à l'atome d'oxygène qui relie l'atome de phosphore au système aromatique ou avec un système aromatique annelé en position méta par rapport à l'atome d'oxygène qui lie l'atome de phosphore au système aromatique, où le radical aromatique en position ortho par rapport à l'atome d'oxygène qui lie l'atome de phosphore au système aromatique, porte un atome d'hydrogène,
R³ : signifiant un radical aromatique avec un radical n-propyle, i-propyle, n-butyle, i-butyle, s-butyle, n-pentyle ainsi que ses isomères, un radical n-hexyle ainsi que ses isomères, un radical cyclopentyle ou cyclohexyle, où les radicaux alkyle cycliques peuvent porter des radicaux alkyle linéaires ou d'autres radicaux alkyle cycliques ou des radicaux aromatiques et les radicaux alkyle peuvent porter des radicaux alkyle cycliques ou des radicaux aromatiques comme substituants, en position para par rapport à l'atome d'oxygène qui lie l'atome de phosphore au système aromatique, ou avec un substituant aromatique en position para par rapport à l'atome d'oxygène qui lie l'atome de phosphore au système aromatique, où le radical aromatique en position ortho par rapport à l'atome d'oxygène qui lie l'atome de phosphore au système aromatique porte un atome d'hydrogène,
R⁴ : signifiant un radical aromatique qui porte, en position ortho, méta et para par rapport à l'atome d'oxygène qui lie l'atome de phosphore au système aromatique, d'autres substituants que ceux définis pour R², R² et R³, où le radical aromatique en position ortho par rapport à l'atome d'oxygène qui lie l'atome de phosphore au système aromatique, porte un atome d'hydrogène, et où, pour a) et b) :
x : vaut 1 ou 2,
y, z, p : indépendamment l'un de l'autre, valent 0, 1 ou 2, à condition que x + y + z + p = 3.

2. Procédé selon la revendication 1, où on utilise un phosphite de formule I avec p = 0.

3. Procédé selon la revendication 1 à 2, où on utilise un phosphite de formule I, où les radicaux R¹, R², R³ et R⁴ sont choisis, indépendamment l'un de l'autre, dans le groupe constitué par un radical naphtyle et un radical phényle substitué selon la revendication 1 ou non substitué.

4. Procédé selon les revendications 1 à 3, où on utilise un phosphite de formule I, où les radicaux R¹, R², R³ et R⁴ sont des radicaux phényle substitués ou non substitués.

5. Complexes de métaux de transition pouvant être obtenus selon les revendications 1 à 4, à condition que y et z représentent 1 ou 2 et p = 0.

6. Utilisation de complexes de métaux de transition selon la revendication 5 comme catalyseur.

7. Utilisation selon la revendication 6 comme catalyseur pour l'addition d'acide cyanhydrique sur une double liaison oléfinique.

8. Utilisation selon la revendication 6 comme catalyseur pour l'isomérisation de nitriles organiques.

9. Procédé pour l'addition d'acide cyanhydrique sur une double liaison oléfinique en présence d'un catalyseur, où on utilise comme catalyseur un complexe de métal de transition selon la revendication 5.

10. Procédé selon la revendication 9, où on additionne de l'acide cyanhydrique sur du butadiène avec obtention d'un composé choisi dans le groupe constitué par le 2-méthyl-3-butènenitrile et le 3-pentènenitrile.

11. Procédé pour l'isomérisation de nitriles organiques en présence d'un catalyseur, où on utilise comme catalyseur un complexe de métal de transition selon la revendication 5.

12. Procédé selon la revendication 11, où on isomérise du 2-méthyl-3-butènenitrile en 3-pentènenitrile.
